# EUROPEAN PATENT APPLICATION

(11) **EP 3 821 979 A1**
(43) Date of publication of application: **19.05.2021**
(21) Application number: 19209631.1
(22) Date of filing: 18.11.2019
(51) Int. Cl.: B01L 3/00, C12M 3/00, C12M 1/00, C12N 15/10, B01D 15/08

(54) **ENHANCED PROCESS FOR SORTING CELLS WITH A MICROFABRICATED VALVE**

(71) Applicant: Miltenyi Biotec B.V. & Co. KG, 51429 Bergisch Gladbach (DE)
(72) Inventor: The designation of the inventor has not yet been filed
(74) Representative: Kisters, Michael Marcus

(57) **Abstract**

The invention is directed to a process for sorting target cells and non-target cells from a suspension by a cell sorting valve microfabricated on a surface of a silicon substrate (10), wherein the cell sorting valve separates the target cells from non-target cells characterized in that the prior to separating the target cells from non-target cells, the suspension is directed over a glass substrate (1500).

## Description

This invention relates to a process for sorting cells in a suspension using a microfabricated, movable cell sorting mechanism where clogging of the sorting mechanism is prevented by removing DNA from the suspension.

### BACKGROUND

Microelectromechanical systems (MEMS) are very small, often moveable structures made on a substrate using surface or bulk lithographic processing techniques, such as those used to manufacture semiconductor devices. MEMS devices may be moveable actuators, sensors, valves, pistons, or switches, for example, with characteristic dimensions of a few microns to hundreds of microns. A moveable MEMS switch, for example, may be used to connect one or more input terminals to one or more output terminals, all microfabricated on a substrate. The actuation means for the moveable switch may be thermal, piezoelectric, electrostatic, or magnetic, for example. MEMS devices can also be made which manipulate particles passing by the MEMS device in a fluid stream.

For example, a MEMS device may be a movable valve, used as a sorting mechanism for sorting various particles from a fluid stream, such as cells from blood. The particles may be transported to the sorting device within the fluid stream enclosed in a microchannel, which flows under pressure. Upon reaching the MEMS sorting device, the sorting device directs the particles of interest such as a blood stem cell, to a separate receptacle, and directs the remainder of the fluid stream to a waste receptacle.

A typical use for a MEMS device is sorting of target form non-target cells out of a cell suspension. Cell suspensions often contain debris of cells, especially DNA and RNA which first enhance the viscosity of the suspension second adhere to the surfaces of the narrow channels of the MEMS device. Any adhered molecule reduces the flow of fluid and in worst case clog the channels of the MEMS device entirely.

In order to prevent clogging of MEMS devices due to adhered DNA, WO2015/132319A1 discloses adding a nuclease to the cell suspension. The nuclease digested DNA and RNA into smaller fragments, which have a reduced tendency to clog the channels and/or enhance the viscosity of the cell suspension. However, adding an enzyme a cell suspension is not always desired as the cells itself might be affected and/or the nuclease prevents further analytic methods downstream of the sorting process.

### Object of the invention

Object of the invention was to prevent clogging of passageways in a MEMS chip during a cell sorting process. In this respect, it was found that the viscosity of the sample can be significant reduced by providing the cell suspension over a glass surface prior to the sorting process. Without being bound to this theory, it is assumed that polar molecules like DNA or oligo nuclides adhere to the glass surface and are thus removed from the cell suspension.

Accordingly, object of the invention is a process for sorting target cells and non-target cells from a suspension by a cell sorting valve microfabricated on a surface of a silicon substrate, wherein the cell sorting valve separates the target cells from non-target cells characterized in that the prior to separating the target cells from non-target cells, the suspension is directed over a glass substrate.

The sorting mechanism used in the method of the invention may be a MEMS fluid valve formed on a silicon substrate, which is adhered to an interposer and installed in a disposable cartridge. The cartridge may provide all the fluidic passageways for the handling of the sample fluid, and may include larger reservoirs (e.g. sort, sample and waste reservoirs) for the storage of volumes of fluids. A plastic interposer is then used to provide the interconnections between the microscopic passages of the MEMS fluid valve and the macroscopic features of the reservoirs. The MEMS fluid valve, interposer and reservoirs may all be contained in a disposable cartridge, such that sterilization of the cell sorting system is straightforward, the cartridge is simply disposed of. Such a cartridge is disclosed in WO2015/132319A1.

A specially designed electromagnet may provide the precisely located electromagnetic fields which cause the very small MEMS chip to move within the much larger system. This electromagnet minimizes heat produced, and thus improves efficiency.

The process may use a cell sorting system which further includes an electromagnet with a tapered tip, coils and magnetic core, wherein the tapered shape serves to concentrate the lines of flux produced by the coils and core, and exit from the electromagnet in the vicinity of the tip.

A MEMS device having these features is disclosed in WO 2015/132319A1.

### BRIEF DESCRIPTION OF THE DRAWINGS

Fig. 1 is a schematic illustration of a MEMS chip sorter in a first position;
Fig. 2 is a schematic illustration of a MEMS chip sorter in a second position; Fig. 3 is a schematic illustration of an exemplary MEMS cell sorting system which may make use of the MEMS sorter of Figs. 1 and 2
Fig. 4 and 5 are exploded view of an exemplary disposable cartridge which may be used in the MEMS cell sorting system of Fig. 3, which includes a MEMS chip sorter and an interposer;

It should be understood that the drawings are not necessarily to scale, and that like numbers may refer to like features.

### DETAILED DESCRIPTION

A method is described for sorting target particles from non-target materials in a fluid stream, using a microfabricated (MEMS) movable valve or sorting mechanism, which directs the target particle from a sample input passageway into a sort passageway, while allowing non-target material to flow into a waste passageway. Both the sort and waste passageways lead to a separate, respective reservoir, the sort and the waste reservoir, and are stored there until removal. The sort, sample and waste reservoirs, along with the MEMS chip sorter, may be contained in a plastic disposable cartridge. This cartridge may then be discarded after the fluids are collected from the reservoirs. This allows greatly reduced burden for sterilizing the system between samples. The systems and methods may also have significant advantages in terms of cost, performance, speed and complexity. The system may also be substantially gentler in its handling of cells, such that viability of cells in the effluent is greatly improved compared to droplet-based flow cytometers.

Because of the microfluidic nature of this cell sorting system, measures are taken to reduce or eliminate clogging, and to handle the small volumes of fluids, and to control the very small movable valve.

### GLASS SUBSTRATE

When using a microfluidic device, the tendency of the cells to agglomerate, clot or clog in the channels may imperil the long-term functionality of the device. This is especially true of biological material, such as blood or cellular suspensions, in which the component particles have a strong tendency to coagulate. The tendency of the material to adhere to the surfaces of the small channels may also cause an undesired, and possibly irreversible, change to the device. The unwanted changes include an increase in subsequent fluidic resistance, contamination of any subsequent sample introduced into the device, and a change in the flow patterns within the device. Even small amounts of nucleic acids in the solution may increase viscosity or form a span of material in such a manner that the channels became clogged and cells within the channels cannot be transported to their destination. As disclosed here, this outcome may be reduced or ameliorated using glass surfaces prior to the sorting process / upstream of the sorting valve.

Preferable, the glass substrate is provided as glass beads having a mean diameter (dso) of 50 to 1000 µm or as glass frit or glass sieve. The glass frit or glass sieve should have a porosity similar to the porosity which is obtained by a bulk of glass beads. More preferred, the glass beads have a mean diameter (dso) of 200-300 µm.

The nature of the glass is not of importance and any type of glass may be used in the process of the invention.

The glass beads are preferable provided as bulk. To remove as much oligo nucleotides as possible, the glass substrate should provide a surface area as large as possible. To this end, the amount of glass beads or the thickness of the glass frit / glass sieve needs to be adjusted accordingly, which is in the knowledge of the person skilled in the art. For example, a volume of glass beads approximately 10 - 50% of the volume of the cell suspension was sufficient to remove enough oligonucleotides to prevent clogging of the MEMS devise/valve.

### PROCESS OF THE INVENTION

In the process of the invention, the suspension is directed over a glass substrate or through a bulk of glass beads. While this may be achieved by simple flow under gravitational conditions, pumping of the suspension is possible too. Aim of the process is to prevent clogging of a microfluidic device due to oligonucleotides adhering to the microfluidic channels or the MEMS valve. "Clogging" does not necessarily mean complete blocking of the microfluidic pathways but reducing the speed of flow to an extend that the sorting process of cells i.e. separating the target cells from non-target cells is slowed down considerable.

Accordingly, the process of the invention is preferable used for suspensions contains oligonucleotides (or suspensions which may contain oligonucleotides) which are then removed at least in part from the suspension prior to separating the target cells from non-target cells by directing the suspension over the glass substrate. While it is preferred to remove all oligonucleotides from the suspension, complete removal is not technically necessary. In praxis, removal of 50 - 90% of the oligonucleotides proved to be sufficient to prevent clogging of the microfluidic pathways.

The process of the invention is preferable performed by providing the suspension to the cell sorting valve via microfabricated channels from a sample reservoir to a sort reservoir and a waste reservoir, wherein the cell sorting valve, sample reservoir, sort reservoir, microfabricated channels and waste reservoir are located in a disposable cartridge.

The process of the invention may include further process steps including but not limited to manipulation, separation, cultivation, lysis, or further analysis of the target cells (or non-target cells if a depletion is performed).

In a first embodiment of such additional downstream process steps, the suspension is provided with magnetic beads prior to separating the target cells from non-target cells.

In a second embodiment of such additional downstream process steps, the target cells and/or the non-target cells are lysed after separation from the suspension.

In a third embodiment of such additional downstream process steps, the target cells and/or the non-target cells are cultivated after separation from the suspension.

In a forth embodiment of such additional downstream process steps, after separating the target cells from non-target cells, the target cells are directed into an aqueous drop within a fluid immiscible with water.

### Cell sorter / MEMS chip

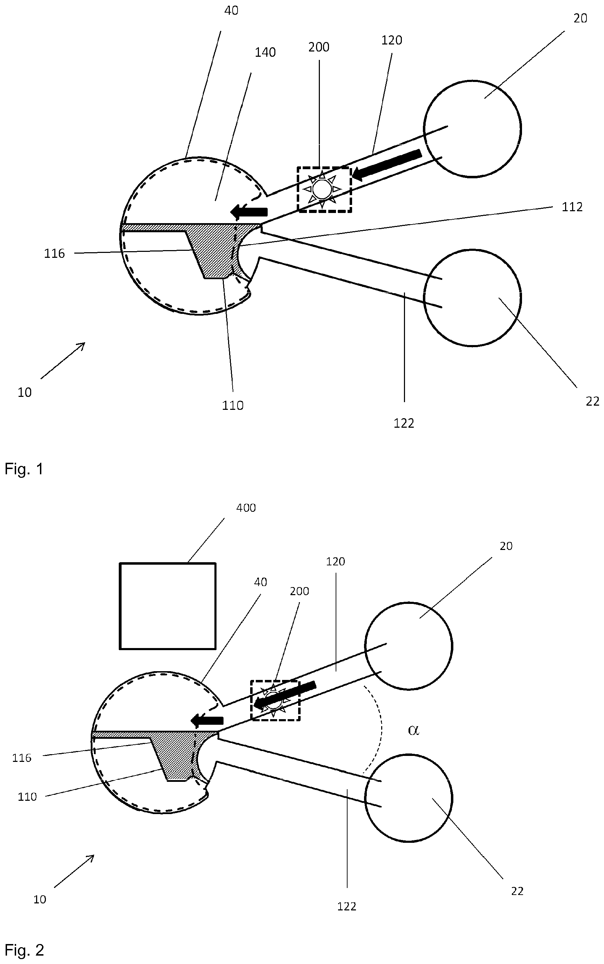

Fig. 1 is a schematic diagram of a microfabricated cell sorting mechanism, MEMS chip sorter 10, which may be used in the particle sorting process described here. Details of cell sorting mechanism may be found in U.S. Patent Application Serial No. 13/998,095 or WO2015132319A1. Among the features of microfabricated cell sorting mechanism 10 is that the motion of the cell sorting valve 10 is parallel to the fabrication plane of the valve. In addition, the waste channel 140 is substantially orthogonal to the sample inlet channel 120 and the sort output channel 122. These features enable distinct advantages in terms of speed and precision, valve throughput and ease of the microfluidic sorting.

It should be understood that the term "chip sorter"10 is an abbreviated term for a microfabricated cell sorting valve 10, as a "chip" is a device microfabricated on a substrate. Either term is intended to designate a microfabricated movable valve formed on a surface of a substrate, using MEMS fabrication techniques, which, by its movement, is capable of separating target particles from non-target material.

In the plan view illustration of Fig. 1, the MEMS chip sorter or cell sorting valve 10 is in the quiescent (un-actuated) position. The chip sorter 10 may include a microfabricated fluidic valve or movable member 110 (hatched area) and a number of microfabricated fluidic channels 120, 122 and 140. Microfabricated fluidic channel 140 (shown as dashed area 140 in Fig. 1 and Fig. 2) serves as output channel and is may be located directly below at least a portion of the microfabricated member 110 and is not parallel to the plane of the microfabricated fluidic channels 120, 122 or the microfabricated member 110. Microfabricated member 110 is fabricated and moves in a path parallel or within this plane. Preferably, the microfabricated fluidic channel 140 is orthogonal to the plane of the microfabricated fluidic channels 120, 122 and the path of motion of microfabricated member 110. The aperture of microfabricated fluidic channel 140 may cover preferably overlap at least a portion of the path of motion of microfabricated member 110, i.e. the dashed, area overlaps the microfabricated member 110 over at least a portion of its motion, as shown in Fig. 1 and Fig. 2. This overlap may allow a fluid path to exist between the input channel 120 and the output channel 140 when the microfabricated member is in the "waste" or unactuated position (Fig. 1), and this path is closed off and the particles redirected in the "sort" or actuated position (Fig. 2). As described previously, this architecture may reduce the fluid resistance, thereby increasing the speed of microfabricated member 110.

The movable member 110 and microfabricated fluidic channels 120, 122 and 140 may be formed on the surface of a suitable substrate, such as a silicon substrate, using MEMS lithographic fabrication techniques as described in greater detail in the '095 application. The fabrication substrate may have a fabrication plane in which the device is formed and in which the movable member 110 moves.

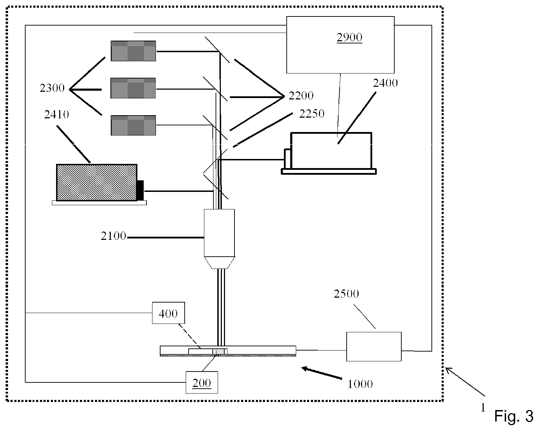

A sample stream may be introduced to the microfabricated movable member 110 by a sample inlet channel 120. The sample fluid may be stored in a sample reservoir 20 prior to sorting by movable member 110. The sample stream may contain a mixture of particles, including at least one desired, target particle and a number of other undesired, non-target particles. The particles may be suspended in a fluid. For example, the target particle may be a biological material such as a stem cell, a cancer cell, a zygote, a protein, a T-cell, a bacterium, a component of blood, a DNA fragment, for example, suspended in a buffer fluid such as saline, or the novel chemistry described below. The inlet channel 120 may be formed in the same fabrication plane as the movable member 110, such that the flow of the fluid is substantially in that plane. The motion of the cell sorting valve 10 is also within this fabrication plane. The decision to sort/save or dispose/waste a given particle may be based on any number of distinguishing signals. In one exemplary embodiment, the decision is based on a fluorescence signal emitted by the particle, based on a fluorescent tag affixed to the particle and excited by an illuminating laser. Laser interrogation region 200 is the portion of the microfluidic passageway in which an illuminating or interrogating laser is directed on the target particle, to distinguish it from the other constituents of the fluid sample. Details as to this detection mechanism are well known in the literature, and further discussed below with respect to Fig. 3. However, other sorts of distinguishing signals may be anticipated, including scattered light or side scattered light which may be based on the morphology of a particle, or any number of mechanical, chemical, electric or magnetic effects that can identify a particle as being either a target particle, and thus sorted or saved, or a non-target particle and thus rejected or otherwise disposed of.

With the movable member 110 in the position shown, the input stream passes unimpeded to a waste output channel 140 which is out of the plane of the inlet channel 120, and thus out of the fabrication plane of the MEMS chip sorter 10. That is, the flow is from the inlet channel 120 to the output orifice 140, from which it flows substantially vertically, and thus orthogonally with respect to the inlet channel 120. This output orifice 140 leads to an out-of-plane channel that may be perpendicular to the plane of the paper showing Fig. 1. More generally, the waste output channel 140 is not parallel to at least one of the plane of the inlet channel 120 or sort channel 122, or the fabrication plane of the movable member 110. In one embodiment, the sort and sample channels may be antiparallel, that is, flow in the sort channel is in an opposite direction to flow in the incoming sample channel.

Accordingly, the cell sorting system may include a cell sorting valve 10, which directs the target particles from a sample channel 120 into a sort channel 122 formed in the silicon substrate and the non-target material from the sample channel 120 to a waste output channel 140 also formed in the silicon substrate. The cell sorting valve 10 may also move in a plane parallel to the surface, and direct the target particles from the sample channel 120 into the waste channel 140 when the microfabricated cell sorting valve 10 is in a first position, and which directs the other particles into the sort channel 122 when in a second position, wherein the sort channel 122 and the waste channel 140 are substantially antiparallel, and the sample channel 120 and waste channel 140 are substantially orthogonal. The waste output channel 140 may have an orifice, which may be a hole formed in the fabrication substrate, or in a covering substrate that is bonded to the fabrication substrate. Further, the movable member 110 may have a curved diverting surface 112 which can redirect the flow of the input stream into a sort output stream. The contour of the surface 112 may be such that redirects the sample stream from the inlet channel 120 into the sort channel 122 in one position, while allowing it to flow to the waste output channel 140 in another position. Accordingly, by having the surface 112 overlap the inlet channel 120, a route exists for the input stream to flow directly into the waste output channel 140 when the movable member 110 is in the un-actuated waste position, as is shown in Fig. 1. The waste output channel 140 may lead to a waste reservoir 40, which may collect the non-target material. The inlaid magnetically permeable material 116 on the movable member 110 may cause its movement, and will be described below in the description of Fig. 2.

Fig. 2 is a plan view of the MEMS chip sorter 10 in the actuated position. In this position, the movable member 110 or valve 10 is deflected upward into the position shown in Fig. 2. The curved diverting surface 112 is a sorting contour which redirects the flow of the inlet channel 120 into the sort output channel 122. The output channel 122 may lie in substantially the same plane as the inlet channel 120, such that the flow within the sort channel 122 is also in substantially the same plane as the flow within the inlet channel 120. There may be an angle a between the inlet channel 120 and the sort channel 122. This angle may be any value up to about 90 degrees. Actuation of movable member 110 may arise from a force from force-generating apparatus 400, shown generically in Fig. 2. In some embodiments, force-generating apparatus 400 may be an electromagnet, as described above. However, force-generating apparatus may also be electrostatic, piezoelectric, or some other means to exert a force on movable member 110, causing it to move from a first position (Fig. 1) to a second position (Fig. 2). The sort channel 122 may lead to a sort reservoir 22 which collects the sorted, target particles as effluent from the movable valve in the position shown in Fig. 2. The inlet channel 120 may conduct the sample fluid from a sample reservoir 20 to the waste channel 140 and waste reservoir 40 as was shown in Fig. 1, or to the sort channel 122 and sort reservoir 22, as shown in Fig. 2.

In some embodiments, the force generating apparatus 400 may include coils which generate a magnetic field, which then interacts with the movable member 110. In order to make the movable member 110 responsive to such an electromagnetic force, it may have a magnetically permeable material inlaid into movable valve 110. The extent of this inlaid magnetic material 116 may be just inside the edge of the outline of the movable member 110 as shown by the dashed lines in Figs. 1 and 2.

A magnetically permeable material should be understood to mean any material which is capable of supporting the formation of a magnetic field within itself. In other words, the permeability of a material is the degree of magnetization that the material obtains in response to an applied magnetic field.

### Cell Detection

In the process, according to the invention the suspension may be provided to the cell sorting valve by a sample channel and the sample channel comprises an interrogation means which distinguishes target particles from non-target materials in the sample stream. Preferable, the interrogation means comprises a laser in a laser-based, induced fluorescence system, wherein a fluorescent tag is affixed to the target particle, and emits a fluorescent signal when irradiated by the laser.

Fig. 3 is a schematic illustration of the cell sorting system 1 which may use microfluidic passageways, a MEMS chip sorter 10 housed in a disposable cartridge 1000, and a flux-generating apparatus 400. What follows is a description of some other components of the system and how they interact with the MEMS chip sorter 10. In particular, Fig. 3 lays out the optical path of the interrogating laser for interrogation region 200, and the control of fluid flow in channels 120-140 and control of MEMS chip sorter 10. After the system level description, the discussion will turn to the unique features of system 1 that allow the microfluidic system 1 to work in a precise, reliable and predictable way.

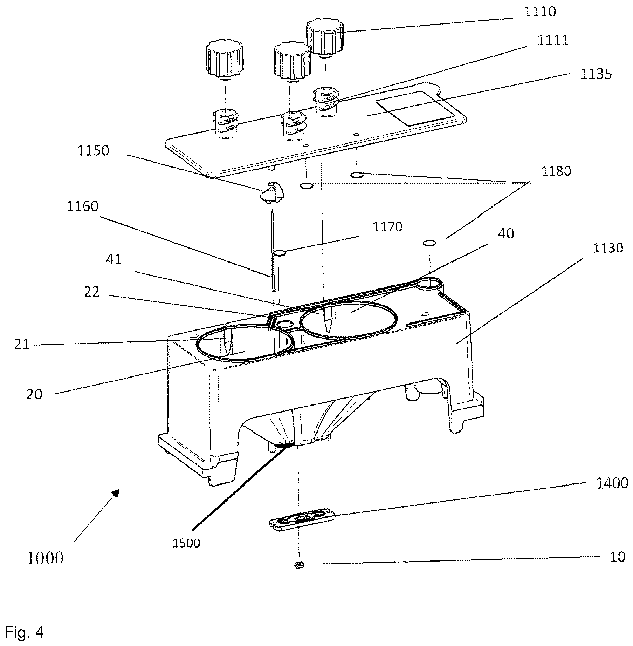

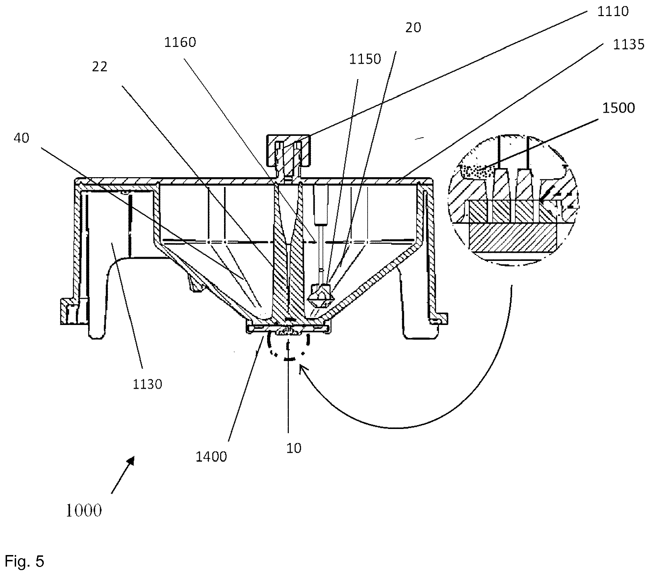

As shown in Fig. 3, the microfabricated MEMS chip sorter 10 may be housed in a disposable cartridge 1000, which may be loaded onto a movable stage and oriented with respect to detection optics 2100 and interrogating lasers 2400 in the cell sorting system 1. Fluid then flows through the MEMS chip sorter 10 from fluid reservoirs also housed in disposable cartridge 1000 through a series of passageways as will be described below with respect to Figs. 4-9.

In the normal operation of system 1, the target particle may be a particular cell, such as a stem cell, or a cancer cell, which has been tagged with a fluorescent marker. This marker emits photons having a particular energy when irradiated with a laser 2400 operating at a predefined wavelength. Accordingly, in this cell sorting system, a laser source 2400 may be directed by a turning mirror 2250 through the detection/collection optics 2100 to the laser interrogation region 200 that was shown in Figs. 1 and 2. The optical axis of the detection/collection optics 2100 and the laser source 2400 may be collinear, at least over a portion of the optical path. Thus, the orientation of the laser application and optical detection along this optical axis may be perpendicular or orthogonal to the substrate fabrication plane, orthogonal to the plane of motion of the movable valve 110 and orthogonal to the flow of the sample fluid through the detection region.

The fluorescence emitted from the irradiated particles may be shaped by detection/collection optics 2100 and separated by dichroic mirrors 2200 and directed into a bank of photodetectors 2300. A plurality of photodetectors may accommodate multiple wavelengths of emitted light, for multiparametric detection. The signal output by the photodetectors 2300 indicates the presence or absence of the target particle in the laser interrogation region 200. The signal may be delivered to a controller 2900, which manages the relative timing of the components in the particle sorting system 1, and collects the data. The controller 2900 may be a general purpose computer or a specialized circuit or ASIC. Upon detection of the target particle, a signal is generated by the controller 2900 which energizes the force-generating or flux-generating apparatus 400. The controller 2900 may also provide the fluidic control to the MEMS chip sorter 10, via one or more pneumatic, hydraulic, piston-based or mechanical force- based mechanisms which are illustrated generically by fluid control means 2500. The rate at which particles are detected may be monitored by the controller 2900, which may maintain the fluid control means 2500.

The force generating apparatus 400 is a device which causes a force to arise in the movable member 110 itself, causing the motion of the movable member. This force-generating apparatus 400 may not be directly mechanically coupled to the MEMS particle manipulation device 10, as indicated by the dashed line in Fig. 3. For example, the force-generating apparatus 400 may be a source of magnetic flux which causes a magnetostatic force to arise in an inlaid permeable material 116 in the MEMS movable member 110 of the cell sorting valve 10 as described previously. Accordingly, flux generating apparatus 400 may be an electromagnet with a magnetic core and windings. This force may pull the movable member 110 toward the force-generating apparatus 400, opening the sort channel 122 and closing the waste channel 140, as was shown in Figs. 1 and 2. Importantly, the force-generating apparatus 400 may reside in the particle sorting system 1, rather than in the MEMS chip sorter 10. As mentioned previously, this may reduce the cost and complexity of the MEMS chip sorter 10, which may be housed in the disposable portion 1000 of the system 1. In the compact system shown in Fig. 3, it is important that excessive heat not be generated by force-generating apparatus 400. As mentioned previously, because of the very small size of MEMS chip sorter 10, force-generating apparatus 400 may also need to generate lines of flux which are concentrated in a small area. Details as to the design of a novel flux-generating apparatus 400 which may be suitable in this application are discussed below with respect to Figs. 10a-10c.

Another optional laser 2410 may also be included to provide a second optical channel in cell sorting system 1.

As mentioned, laser interrogation region 200 is the portion of the microfluidic passageway in which the laser 2400 is directed on the target particle, in order to distinguish it from the other constituents of the fluid sample.

Upon passing through the detection region 200, a signal is generated by the detector 2300 indicating that a target particle is present in the interrogation region 200. After a known delay, a signal is generated by the controller 2900 which indicates that the sorting gate, i.e. the movable member 110 of the cell sorting valve10 is to be opened, in order to separate the target particle which was detected, from the other components in the fluid stream. The movable member 110, of the MEMS valve 10 may comprise permeable magnetic materials 116 as mentioned previously, so that the magnetic force may arise in it in the presence of a magnetic field. When the signal is generated by the controller 2900, a force arises in the embedded magnetically permeable material 116 which draws the movable valve 110 toward the force generating apparatus 400. This motion may close off waste channel 140 and redirect the target particle into a sort channel 122. The sorted sample is subsequently collected from a sort reservoir at the end of the sort channel 122, which holds the sorted sample. As mentioned previously, the controller 2900 may also control flow rates based on the rate at which sorting events are recorded.

### Disposable cartridge as sample reservoir

Fig. 4 is an exploded perspective view of an exemplary disposable cartridge 1000 which may be used in the particle sorting system shown in Fig. 3. Disposable cartridge 1000 may include several pieces, such as top 1135 and base 1130.

Disposable cartridge 1000 may house MEMS chip sorter 10 and provide storage in fluid reservoirs. Accordingly, the base 1130 of disposable cartridge 1000 may have a plurality of voids or compartments formed therein, including sample reservoir 20, sort reservoir 22 and waste reservoir 40. As described further below, the sample to be sorted may be stored in sample reservoir 20, the sort effluent in sort reservoir 22 and waste effluent in waste reservoir 40. The fluidic passageways between these voids may all be disposed in the interposer 1400 and/or in the MEMS chip sorter 10. Accordingly, the interposer 1400 may provide a sort fluid path between a sort reservoir 22 in the disposable cartridge and the sort channel 122 in the silicon substrate, a waste fluid path between a waste reservoir 40 in the disposable cartridge and the waste channel 140, and a sample fluid path between the sample channel 120 and a sample reservoir 20.

It should be understood that the term "sort" fluid, "sort" sample or "sort" reservoir may refer to a collection of target particles. The "waste" fluid, "waste" sample or "waste" reservoir may refer to a collection of non-target materials in the fluid stream. Other equivalent language is "positive fraction" to refer to the sort sample, and "negative fraction" to refer to non-target material. Accordingly, in the text below, "sort" portion may be equivalent to the "positive fraction" and refer to a collection of target particles, and "waste" may refer to the negative fraction and to a collection of non-target materials.

The glass substrate should be located upstream of any microfabricated pathway i.e. at least upstream of the cell sorting valve microfabricated on a surface of a silicon. As shown in Fig 3 and 4, this may be achieved by locating a glass frit or glass sieve or bulk of glass beads (1500) at the lowermost point of the reservoir. This bulk may be placed on and/or covered by a filter means as support or to prevent the beads from dislocation. As filter means, any porous material of mesh can be used. If a bulk of glass beads is used as glass substrate, a glass frit or glass sieve may be used as filter means.

In alternative, a glass frit or glass sieve or bulk of glass beads may be provided as part of the interposer (1400) or as additional layer between the interposer 1400 and the MEMS chip 10.

### EXAMPEL

The DNA-binding effect of glass beads has been shown in an experiment using 2 day old lysed whole blood drawn. The cells were stained with CD45Vioblue and CD3 PE and final input concentration for the experiment was ∼ 2 million cells/mL at 98% viability by PI.

For the experiment the cells were loaded into a cartridge of the MACSQuant Tyto machine (obtainable from Miltenyi Biotec B.V. & Co. KG, in the following referred to as "Tyto") and sample analysis was initiated (Trigger on CD45 Vioblue and useCD3 PE for flow control).

In total three different conditions were tested:
1. Standard Tyto cartridge (3D filter/11 um mesh) with the sample in standard Tyto Running Buffer comprising Benzonase.
2. Standard Tyto cartridge (3D filter/11 um mesh) with the sample in Part A Tyto Running Buffer without the addition of Benzonase.
3. Cartridge with the 3D filter removed and glass beads added to the same height as the 3D filter with sample in Part A Tyto Running Buffer without the addition of Benzonase. The glass beads had a mean diameter of 200 µm.

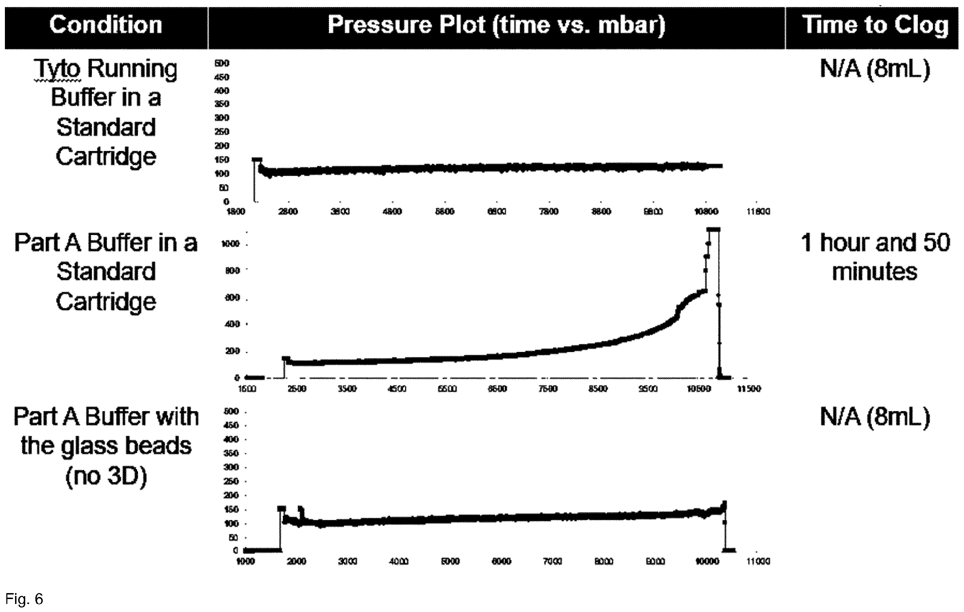

As read-outs following parameters were used: time to clog, pressure plots and PI stain of the filter after sorting. Fig. 6 shows the results of this experiment: whereas the cartridge in run 2 (Tyto Running Buffer without the addition of Benzonase) clogs after 1h50 min indicated by the sharp increase of pressure, in run 1 and 3, no clogging was observed.

The pressure development in case of run 3 (without Benzonase, with glass beads) was comparable to the pressure course of condition 1 (Tyto Running Buffer with Benzonase) while the standard cartridge without Benzonase (condition 2) did clog demonstrating that the sample was sticky enough to cause issues. Consistent with this finding, the PI staining after the sort showed that some DNA debris did bind to the glass beads.

The experiment was repeated with glass beads having a mean diameter of 75 - 90 µm with similar results.

Accordingly, the process of the invention performed without the addition of Benzonase as good as a process using the standard Tyto Running Buffer comprising Benzonase.

## Claims

1. Process for sorting target cells and non-target cells from a suspension by a cell sorting valve microfabricated on a surface of a silicon substrate, wherein the cell sorting valve separates the target cells from non-target cells **characterized in that** the prior to separating the target cells from non-target cells, the suspension is directed over a glass substrate.

2. Process according to claim 1 **characterized in that** the glass substrate is provided as glass beads having a mean diameter of 50 to 1000 µm or as glass frit or glass sieve.

3. Process according to claim 1 or 2 **characterized in that** the suspension contains oligonucleotides and the is removed at least in part from the suspension prior to separating the target cells from non-target cells by directing the suspension over the glass substrate.

4. Process according to any of the claim 1 to 3 **characterized in that** the suspension is provided to the cell sorting valve via microfabricated channels from a sample reservoir to a sort reservoir and a waste reservoir, wherein the cell sorting valve, sample reservoir, sort reservoir, microfabricated channels and waste reservoir are located in a disposable cartridge.

5. Process according to any of the claim 1 to 4 **characterized in that** the suspension is provided with magnetic beads prior to separating the target cells from non-target cells.

6. Process according to any of the claim 1 to 5 **characterized in that** the target cells and/or the non-target cells are lysed after separation from the suspension.

7. Process according to any of the claim 1 to 6 **characterized in that** the target cells and/or the non-target cells are cultivated after separation from the suspension.

8. Process according to any of the claim 1 to 7 **characterized in that** after separating the target cells from non-target cells, the target cells are directed into an aqueous drops within a fluid immiscible with water

9. Process according to any of the claim 1 to 8, **characterized in that** the suspension is provided to the cell sorting valve by a sample channel and the sample channel comprises an interrogation means which distinguishes target particles from non-target materials in the sample stream.

10. Process according to claim 9, **characterized in that** the interrogation means comprises a laser in a laser-based, induced fluorescence system, wherein a fluorescent tag is affixed to the target particle, and emits a fluorescent signal when irradiated by the laser.
